(19) Europäisches Patentamt
European Patent Office
Office européen des brevets

(11) **EP 4 372 323 A1**

(12) **EUROPEAN PATENT APPLICATION**

(43) Date of publication:
**22.05.2024 Bulletin 2024/21**

(21) Application number: **23210565.0**

(22) Date of filing: **17.11.2023**

(51) International Patent Classification (IPC):
*G01F 1/36* $^{(2006.01)}$  *G01F 1/44* $^{(2006.01)}$
*G01N 11/08* $^{(2006.01)}$  *G01F 1/88* $^{(2006.01)}$
*G01F 15/02* $^{(2006.01)}$  *G01F 1/50* $^{(2006.01)}$
*G01J 5/04* $^{(2006.01)}$  *G01J 5/0875* $^{(2022.01)}$
*A61M 1/14* $^{(2006.01)}$  *A61M 60/37* $^{(2021.01)}$
*G01N 11/00* $^{(2006.01)}$

(52) Cooperative Patent Classification (CPC):
**G01N 11/08; A61M 60/37; G01F 1/36; G01F 1/44;
G01F 1/50; G01F 1/88; G01F 15/024; G01J 5/041;
G01J 5/0875;** G01N 2011/0013

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **18.11.2022 IT 202200023838**

(71) Applicant: **Fluid-O-Tech S.r.l.**
**20094 Corsico (MI) (IT)**

(72) Inventors:
• **ANDREIS, Diego**
**20094 Corsico (MI) (IT)**

• **BRATTO, Andrea**
**80071 Anacapri (NA) (IT)**
• **TESSICINI, Fabrizio**
**40026 Imola (BO) (IT)**
• **BRUNO, Giuseppe**
**95047 Paterno' (CT) (IT)**
• **ALESSI, Enrico**
**95127 Catania (CT) (IT)**

(74) Representative: **Bianchetti & Minoja with
Trevisan & Cuonzo IPS SRL
Via Plinio, 63
20129 Milano (IT)**

(54) **SYSTEM FOR IN-LINE MEASUREMENT OF THE VISCOSITY OF A FLUID AND CORRESPONDING MEASUREMENT METHOD**

(57)     The present invention concerns a system (1) for in-line measurement of the viscosity of a fluid flowing within a duct (3), comprising:
- a duct (3) having three distinct cross-sections (S 1, S2, S3), where said three distinct cross-sections (S1, S2, S3) are distributed along the direction of axial development of said duct (3) and where at least one cross-section (S2), intermediate between two end cross-sections (S1, S3), has a cross-sectional area (A2) of greater or lesser extension than the cross-sectional area (A1, A3) of each of said two end cross-sections (S1, S3);
- at least three pressure sensors (P1, P2, P3) arranged in correspondence with said three distinct cross-sections (S1, S2, S3) of said duct (3) and configured to detect the instantaneous pressure of said fluid in correspondence with said three distinct cross-sections (S1, S2, S3) of said duct (3).

Fig. 1

**Description**

The present invention refers to a system for in-line measurement of the viscosity of a fluid and to the corresponding measurement method.

As is known, the viscosity of a fluid is a physical quantity that measures the sliding resistance. The viscosity of a fluid is measured with viscometers, or with rheometers in case the fluid is of non-homogeneous type.

The viscometers of known type are not free of drawbacks, among which is the fact that they do not allow performing an in-line measurement of the viscosity.

In addition, the measurement of the viscosity is strongly dependent on the measuring instrument used and on the conditions under which the measurement is carried out.

At the current state of the art, the viscosity sensors that can be integrated into simple and economical fluidic systems have parts in relative movement that must be repositioned at every measurement and/or use additional channels parallel with respect to the main channel where the fluid flows. These sensors represent a complication for the systems in which they are installed, both in hydraulic terms and in terms of cleaning. For these reasons, for example, they cannot be integrated into hydraulic systems that convey food or medical liquids.

The main task of the present invention consists in the fact of realising a system for in-line measurement of the viscosity of a fluid, and a corresponding measurement method, which precisely allows to carry out an in-line measurement of the viscosity of the fluid.

Within the scope of this task, an aim of the present invention is to realise a system for in-line measurement of the viscosity of a fluid, and a corresponding measurement method, which is not affected by the conditions under which the measurement is carried out.

Another aim of the invention consists in the fact of realising a system for in-line measurement of the viscosity of a fluid that is easy to realise and to integrate into hydraulic circuits of various types, and that can also be of the disposable type.

A further aim of the invention consists in the fact of realising a system for in-line measurement of the viscosity of a fluid that is capable of giving the widest guarantees of reliability and safety in use.

Yet another aim of the invention consists in the fact of realising a system for in-line measurement of the viscosity of a fluid that also allows the flow of the fluid to be measured regardless of the viscosity of the fluid itself.

The above task, as well as the purposes and other that will appear better hereinafter, are achieved by a system for in-line measurement of the viscosity of a fluid as set forth in claim 1, as well as by a method for in-line measurement of the viscosity of a fluid as set forth in claim 11.

An object of the present invention is also a system for in-line measurement of the flow of a fluid, as set forth in claim 14, as well as a method for in-line measurement of the flow of a fluid as set forth in claim 15.

Other features are provided in the dependent claims.

Further characteristics and advantages will result more from the description of a preferred, but not exclusive, embodiment of a system for in-line measurement of the viscosity of a fluid, illustrated by way of example and not limitation with the aid of the attached drawings in which:

- Figure 1 is a schematic view of an embodiment of a system for in-line measurement of the viscosity of a fluid, according to the invention;
- Figure 2 is a schematic side view of a portion of a duct of the system of Figure 1, where a temperature sensor is located;
- Figure 3 is a schematic sectional view of the duct depicted in Figure 2 according to axis III-III;
- Figure 4 is a schematic side view of a variant of a portion of a duct of the system of Figure 1, where a temperature sensor is located;
- Figure 5 is a schematic sectional view of the duct depicted in Figure 4 according to axis V-V;
- Figure 6 is a schematic view of a system for in-line measurement of the viscosity of a fluid of the disposable type, according to the invention, applied to a hydraulic circuit;
- Figure 7 is a schematic view of a system for measuring the degree of carbonation of water in which there is a system for in-line measurement of the flow of water, according to a variant of the invention.

With reference to the cited figures, the system for in-line measurement of the viscosity of a fluid flowing within a duct is indicated globally with the reference numeral 1.

According to the invention, said system 1 comprises:

- a duct 3 having three distinct cross-sections S1, S2, S3, where said three distinct cross-sections S1, S2, S3 are distributed along the direction of axial development of said duct 3 and where at least one cross-section S2, intermediate between two end cross-sections S1, S3, has a cross-sectional area A2 of greater or lesser extension than the cross-sectional area A1, A3 of each of the two end cross-sections S1, S3;

- at least three pressure sensors P1, P2, P3 arranged in correspondence with said three distinct cross-sections S1, S2, S3 of the duct 3 and configured to detect the instantaneous pressure of the fluid in correspondence with said three distinct cross-sections S1, S2, S3 of the duct 3.

Preferably the three pressure sensors P1, P2 and P3 are absolute pressure sensors. Alternatively, the three pressure sensors P1, P2 and P3 may be differential pressure sensors.

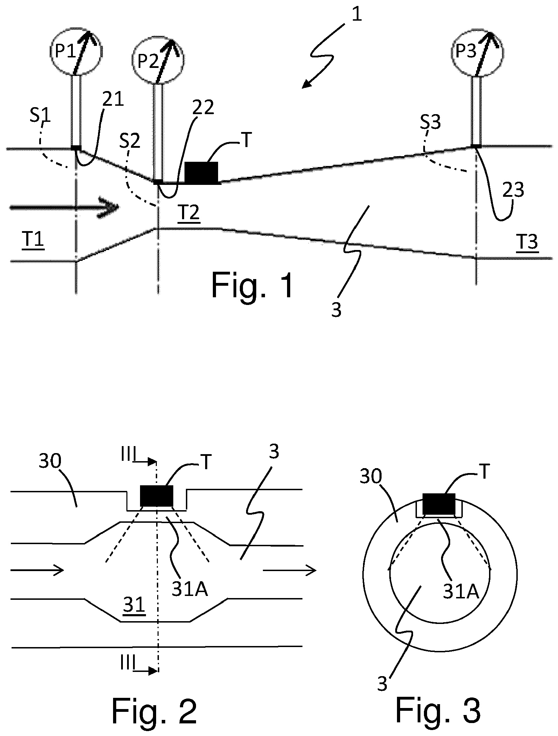

Preferably, as illustrated in the example of Figure 1, the intermediate cross-section S2 has a cross-sectional area A2 of smaller extension than the cross-sectional areas A1, A3 of each of the two end cross-sections S1, S3.

The intermediate cross-section S2 may consist of a constriction of the duct 3. This constriction can be obtained symmetrically in the centre of the duct 3 itself, as illustrated in the schematic example of Figure 1, or even laterally, asymmetrically, with respect to the longitudinal axis of the duct 3 itself.

The duct 3, and therefore the relative cross-sections S1, S2, S3, can have a circular, square or rectangular shape.

Preferably, the two end cross-sections S1, S3 have cross-sectional areas A1, A3 of equal extension.

The operation of the system 1 for in-line measurement of the viscosity of a fluid is described below.

The fluid flowing in the duct 3 along the direction of axial development of the duct 3 itself passes, in sequence, through the first end cross-section S1, the intermediate cross-section S2, and the second end cross-section S3. The pressure sensors P1, P2 and P3 detect the instantaneous pressure of the fluid in the respective sections S1, S2 and S3.

The fluid enters the section S1 (of known area *A1*) at a certain pressure p1 (measured by the pressure sensor P1) and with a certain speed $v_1$.

Subsequently, the fluid passes through the section S2 (of known area A2, preferably minor than area A1).

Given the variation in section, the fluid, according to the conservation of flow, changes its speed, which becomes $v_2$.

According to Bernoulli's theorem, if the fluid were ideal, its pressure $p_2$ in correspondence with the section S2 will be given by the following equation:

$$p_1 + \frac{1}{2}\rho v_1^2 + \rho g H_1 = p_2 + \frac{1}{2}\rho v_2^2 + \rho g H_2$$

where $\rho$ is the density of the fluid, g is the acceleration of gravity, and $H_1$ and $H_2$ are the average potential height in the section S1 and in the section S2, respectively.

In the case of interest, the following hypotheses also occur:

1) $\rho g H_1 - \rho g H_2 = 0$, since the potential energy difference can be considered negligible compared to the other terms of the equation, or in any case compensated for, as explained below;
2) $Q = A1 * v_1 = A2 * v_2$, according to the law of mass conservation, being: $\rho_1 = \rho_2$, i.e. being p constant along the duct 3.
3) Since it is a viscous fluid, it also occurs that:

$$p_1 + \frac{1}{2}\rho_1 v_1^2 + \rho_1 g H_1 = p_2 + \frac{1}{2}\rho_2 v_2^2 + p_2 g H_2 + \Delta p_{losses}$$

where

$$\Delta p_{losses} = f(\mu, v_1, geometry)$$

that is, where the pressure loss $\Delta p_{losses}$ is a function of the dynamic viscosity of the fluid and of the geometry of the duct 3, fixed a priori.

Consequently, it follows that:

$$p_1 + \frac{1}{2}\rho v_1^2 = p_2 + \frac{1}{2}\rho v_2^2 - \Delta p_{losses}$$

And hence:

$$\Delta p_{1,2} = p_1 - p_2 = \frac{1}{2}\rho\left(1 - \frac{A1}{A2}\right)v_1^2 - \Delta p_{losses}$$

The fluid subsequently passes through the second end cross-section S3, which preferably has a cross-sectional area A3 of extension equal to the cross-sectional area *A1* of the first end cross-section *S1*.

In the ideal case, without pressure losses due to the viscous friction, the pressure $p_3$ in correspondence with the section S3 would return to be the initial one $p_1$. In the real case, however, given the losses due to the viscous friction, the pressure $p_3$ in the section S3 will be different from $p_1$ in the section *S1* by an amount that is proportional to the speed and viscosity of the fluid.

The following hypotheses occurring:

1) A1 = A3
2) $Q = A1 * v_1 = A3 * v_3$, according to mass conservation, being: $\rho_1 = \rho_3$ it will follow that:

$$v_1 = v_3$$

And hence:

$$\Delta p_{1,3} = p_1 - p_3 = \Delta p_{losses} = f(\mu, v_1, geometry)$$

To summarize, the system of equations is given by the following two equations:

$$p_1 - p_2 = \frac{1}{2}\rho\left(1 - \frac{A1}{A2}\right)v_1^2 - \Delta p_{losses}(v_1, \mu) \text{ and } p_1 - p_3 = \Delta p_{losses}(v_1, \mu)$$

In this system, $p_1$, $p_2$ and $p_3$ are measured. The geometry, and therefore *A1* and *A2*, are known. The density of the fluid $\rho$ is known or in any case has small negligible variations due to the composition or the temperature of the fluid.

The only two unknowns of this system of two equations are $v_1$ (therefore indirectly, the flow *Q*) and the viscosity $\mu$ which are therefore uniquely determined.

$$Q = f_1(p_1, p_2, p_3)$$

$$\mu = f_2(p_1, p_2, p_3)$$

Therefore, as demonstrated above, the system 1 allows to measure the viscosity of a fluid starting from the detection of the pressures $p_1$, $p_2$ and $p_3$ which occurs through the three pressure sensors P1, P2 and P3.

Preferably the pressure sensors P1, P2 and P3 are miniaturized sensors, preferably of the MEMS type, from the acronym micro electro-mechanical systems.

Preferably, each of said pressure sensors P1, P2 and P3 comprises a sensing element 21, 22, 23 configured to face the internal volume of the duct 3 and to be in direct contact with the fluid.

Preferably, said sensing element 21, 22, 23 is integrated into the wall 30 of the duct 3 itself and even more preferably positioned flush with the inner face of the wall 30 itself.

Advantageously, the miniaturization of the pressure sensors P1, P2 and P3 and the positioning of the relative sensing elements 21, 22 and 23 flush with the wall 30 and in direct contact with the fluid flowing in the duct 3 allow to guarantee the correct interfacing of the sensors minimizing the dead spaces, so as to make the cleaning of the duct 3 itself possible, according to current international standards. In fact, for example, lateral connection channels adapted to put the duct 3 itself and the pressure sensors in fluid communication are not provided.

Preferably, the pressure signal detected by the pressure sensors P1, P2 and P3 can be acquired by a local control unit that can be integrated into the pressure sensor P1, P2 and P3 itself, to then be sent to a central control unit with which the system 1 is provided, or it can be acquired directly by said central control unit.

As mentioned, the pressure sensors P1, P2 and P3 measure the absolute pressure. Each of them, therefore, will have a certain offset with respect to the others due to the intrinsic calibration error of these sensors. Furthermore, during prolonged use of the pressure sensors P1, P2 and P3, this error may vary.

Preferably, the system 1 is therefore provided with a self-calibration procedure of the pressure sensors P1, P2 and P3 which takes place through the firmware loaded on the central control unit of the system 1 itself.

The purpose of the calibration of the pressure sensors P1, P2, P3 is to align the sensors so as to always provide the real pressure difference due to the Venturi effect alone between the sensors, in any operating condition, thus eliminating any dependence on measurement errors or any differences due to the term of potential energy in the Bernoulli's equation or the deterioration of the sensors over time.

To carry out this calibration, a correction term $\Delta p_{1,2}$, at the firmware level, is added to the calculation of the $\Delta p_{corr}$. In this way, it follows that:

$$\Delta p_{1,2} = p_1 - p_2 + \Delta p_{corr}$$

The sizing of $\Delta p_{corr}$ is done on the basis of Bernoulli's equation:

$$p_1 + \frac{1}{2}\rho v_1^2 + \rho g H_1 = p_2 + \frac{1}{2}\rho v_2^2 + \rho g H_2$$

If it is imposed on the system that the fluid is stationary and therefore that $v_1 = v_2$, it follows that:

$$\Delta p_{1,2\,a\,v=0} = \rho g(H_1 - H_2) + Err_{tot} = \Delta p_{corr}$$

The calibration system can also provide an estimate of this $\Delta p_{corr}$ at various pressures. In this way one will have a variable corrective factor as a function of the operating pressure.

Preferably, as illustrated in the example of Figure 1, the duct 3 has, in sequence:

- at least a first segment T1 having substantially constant cross-section S1;
- at least a second segment T2 having substantially constant cross-section S2;
- at least a third segment T3 having substantially constant cross-section S3.

Advantageously, in this way, the pressure sensors P1, P2 and P3 can be arranged in correspondence with portions of the duct 3 where the fluid has a substantially constant flow as it is negligibly affected by the cross-section variations of the duct 3 in correspondence with the segments with increasing or decreasing cross-section that connect the aforementioned segments T1, T2, and T3 with a constant cross-section.

Preferably, the system 1 also comprises a temperature sensor T configured to detect the instantaneous temperature of the fluid flowing in the duct 3.

Preferably said temperature sensor T is an infrared sensor associated with a wall 30 of the duct 3.

Preferably, said wall 30 comprises at least one portion 31A transparent to infrared rays that is interposed between the temperature sensor T and the internal volume of the duct 3.

As illustrated for example in Figures 2 and 3, the wall 30 of the duct 3 is made, at least in the portion 31A in correspondence with the sensor T, of a material transparent to infrared rays. In this way the temperature sensor T can directly detect the temperature of the fluid flowing within the duct 3 without being in contact with the fluid itself and without the detection of the temperature being influenced by the temperature of the wall 30 itself of the duct 3.

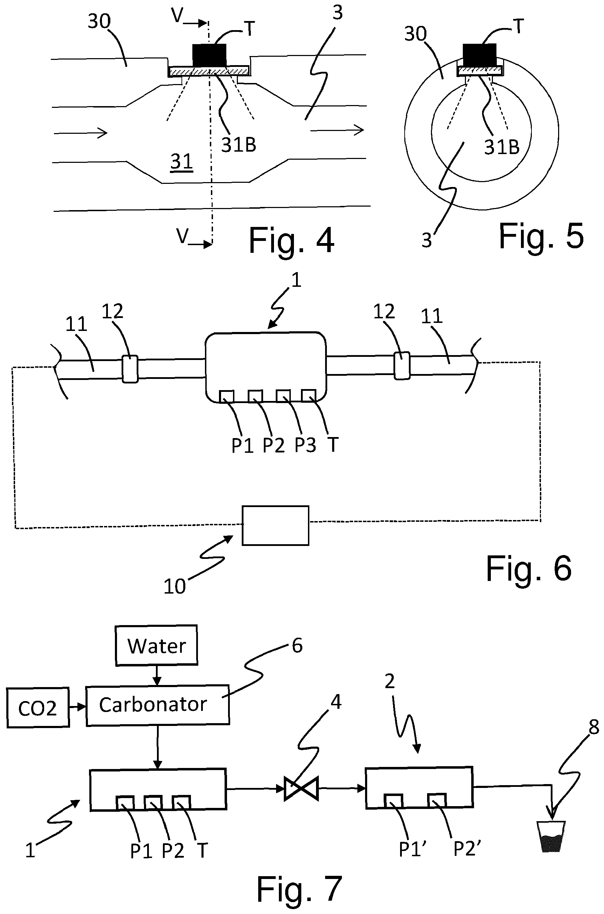

According to the variant illustrated in Figures 4 and 5, the temperature sensor T is always an infrared sensor associated with the wall 30 of the duct 3, but the system 1 comprises at least one body 31B transparent to infrared rays, associated with the wall 30 and interposed between the temperature sensor T and the internal volume of the duct 3. Advantageously, therefore, the wall 30 of the duct 3 can be made of any material, but the detection of the temperature of the fluid is performed through the aforementioned transparent body 31B, which essentially constitutes a transparent window that allows the temperature sensor T to directly detect the temperature of the fluid flowing within the duct 3 without being in contact with the fluid itself and without the detection of the temperature being affected by the temperature of the wall 30 itself of the duct 3.

Preferably the temperature sensor T is a miniaturized sensor, preferably of the MEMS type, from the acronym micro electro-mechanical systems.

Preferably, the duct 3 has, in correspondence with the portion where the temperature sensor T is located, a segment 31 having a cross-section of greater area than the cross-section of the duct 3 in portions upstream and downstream of the segment 31 itself.

Advantageously, the fact of providing a segment 31 with an increased section ensures that the sensing field of the temperature sensor T, depicted in Figures 2-5 by a dashed line, substantially entirely involves the fluid flowing in the duct 3 itself and at most only minimally the walls 30 of the duct 3. In essence, the increased section of the internal volume of the duct 3 in correspondence with the temperature sensor T ensures that the measurement sensor T detects the actual temperature of the fluid and not the temperature of the wall 30 of the duct 3, which could deviate from the temperature of the fluid present in the duct 3.

The present invention also concerns a method for in-line measurement of the viscosity of a fluid flowing within a duct 3, comprising the steps of:

- detecting the instantaneous pressure of the fluid in correspondence with three distinct cross sections S1, S2, S3 of the duct 3 where said three distinct cross sections S1, S2, S3 are distributed along the direction of axial development of the duct 3 and where at least one cross section S2 intermediate between two end cross sections S1, S3 has a cross-sectional area A2 of greater or lesser, preferably lesser, extension than the cross-sectional area A1, A3 of each of the two end cross sections S1, S3;
- calculating the viscosity of the fluid on the basis of the instantaneous pressure of the fluid detected in correspondence with the three distinct cross-sections S1, S2, S3 of the duct 3.

Preferably, said method further comprises the step of detecting the instantaneous temperature of the fluid, for example through the temperature sensor T.

Preferably, as described further above with reference to the system of in-line measurement of the viscosity, said step of calculating the viscosity $\mu$ of the fluid comprises the step of solving the system of equations:

$$p_1 - p_2 = \frac{1}{2}\rho\left(1 - \frac{A1}{A2}\right)v_1^2 - \Delta p_{losses}(v_1, \mu)$$ and $p_1 - p_3 = \Delta p_{losses}(v_1, \mu)$ where

- $p_1$, $p_2$ and $p_3$ are the pressures detected by the pressure sensors P1, P2, P3, respectively,
- $\rho$ is the density of the fluid,
- A1 is the area of each of the two end cross-sections S1, S3 (configured to have the same area),
- A2 is the area of the intermediate cross-section S2,
- $v_1$ is the speed of the fluid in correspondence with each of the two end cross-sections S1, S3, and wherein $\Delta p_{losses}$ = $f(\mu, v_1, geometry)$, i.e. the pressure loss $\Delta p_{losses}$ is a function of the dynamic viscosity $\mu$, of the fluid and of the geometry of the duct 3, where such geometry is known, A1, A2, A3 being known.

The unknowns of the aforementioned system of two equations are therefore $v_1$ and the viscosity $\mu$, which are therefore uniquely determinable by solving the system itself.

Preferably, as described further above, the method for in-line measurement of the viscosity of a fluid comprises the step of performing a self-calibration procedure of the pressure sensors P1, P2, P3 comprising the step of calculating a correction factor $\Delta p_{corr}$ of the pressure difference detected between pairs of such pressure sensors P1, P2, P3 at zero fluid speed (e.g., $\Delta p_{1,2\ a\ v=0} = \rho g(H_1 - H_2) + Err_{tot} = \Delta p_{corr}$).

Preferably, the method for in-line measurement of the viscosity of a fluid comprises the step of autonomously detecting the conditions necessary to be able to perform the aforementioned self-calibration procedure and then the step of performing said self-calibration procedure.

For example, whenever a zero speed of the fluid flow within the duct 3 is detected, the self-calibration procedure is started.

More particularly, for example, in the case where the system for in-line measurement of the viscosity 1 detects an absence of fluctuations of the pressure $p1$, $p2$, $p3$ detected by the pressure sensors P1, P2, P3, for example it detects fluctuations of less than 1%, or even less than 0.01%, of the average pressure value, the self-calibration procedure may be started.

Advantageously, the method also comprises the step of performing a procedure of self-calibration of the temperature sensor T, which can be started upon reaching desired temperature values or upon reaching desired temperature time gradients, measured by the temperature sensor T itself.

Advantageously, the system 1 as described above also allows to measure the flow of the fluid flowing within the duct 3. To do this, in fact, it is sufficient to use only two pressure sensors P1, P2 out of the three available.

Moreover advantageously, the possibility of also detecting the temperature of the fluid makes it possible to measure the flow of fluids whose viscosity changes as a result of variations in temperature, using precisely the temperature signal of the fluid to compensate for the effects due to variations in temperature and therefore in the viscosity of the fluid itself.

In particular, being the viscosity-temperature curve of the fluid flowing within the duct 3 known, it is possible to measure the flow of the fluid by compensating for the effects due to the variation in temperature of the fluid itself.

Finally, the present invention also concerns a system 1 for in-line measurement of the flow of a fluid flowing within a duct 3, comprising:

- a duct 3 having at least two distinct cross-sections S1, S2 distributed along the direction of axial development of the duct 3 and where at least a first cross-section S1 has a cross-sectional area A1 of greater or lesser extension, preferably greater, than the cross-sectional area A2 of the second cross-section S2 downstream of the first cross-section S1;
- at least two pressure sensors P1, P2, preferably of the type of absolute pressure sensors, arranged in correspondence with said two distinct cross-sections S1, S2 of the duct 3 and configured to detect the instantaneous pressure of the fluid in correspondence with the two distinct cross-sections S1, S2 of the duct 3;
- at least one temperature sensor T configured to detect the instantaneous temperature of the fluid flowing in the duct 3.

Preferably, the temperature sensor T and the duct 3 in correspondence with the sensor T itself have the technical characteristics described above with reference to the two variants of the system illustrated in Figures 2-3 and 4-5.

Preferably, also the duct 3, as far as the portions thereof in correspondence with the first two cross-sections S1 and S2 are specifically concerned, has the technical characteristics described above and illustrated in Figure 1.

In other words, the present invention also concerns a system for the measurement of the flow of a fluid which, with respect to the embodiment of the system 1 illustrated in Figure 1, does not necessarily also comprise the pressure sensor P3 and therefore does not have a specific end cross-section S3.

The present invention therefore also concerns a method for in-line measurement of the flow of a fluid flowing within a duct 3, comprising the steps of:

- detecting the instantaneous pressure of the fluid in correspondence with at least two distinct cross-sections S1, S2 of the duct 3 distributed along the direction of axial development of the duct 3, where at least a first cross-section S1 has a cross-sectional area A1 of greater or lesser extension, preferably greater, than the cross-sectional area A2 of the second cross-section S2 downstream of the first cross-section S1;
- detecting the instantaneous temperature of said fluid;
- calculating the flow of the fluid on the basis of the instantaneous pressure detected in correspondence with the two distinct cross-sections S1, S2 of the duct 3 and on the basis of said instantaneous temperature.

Advantageously, the system and the method last described make it possible to measure the flow of fluids whose viscosity changes as a result of variations in temperature, using precisely the temperature signal of the fluid to compensate for the effects due to variations in temperature and therefore in the viscosity of the fluid itself.

Preferably the system 1 as described above, both for the measurement of the viscosity and for the measurement of the flow, can be of the disposable type, i.e. it can be made with pressure sensors P1, P2, P3 and/or with temperature sensors T at low cost.

As illustrated in Figure 6, the system 1 can be integrated at any point of a hydraulic circuit 10, and in particular in correspondence with a segment of a hydraulic duct 11 of said hydraulic circuit 10, by means of two connecting elements 12. The system for in-line measurement of the viscosity 1 is therefore advantageously integrated into a disposable device that can be easily installed in a point of a hydraulic circuit 10 in correspondence with which it is desired to detect and monitor the viscosity and/or the flow of the fluid.

In other words, therefore, the system for in-line measurement of the viscosity 1 comprises a duct 3 which is provided, at its axial ends, with respective fittings 12 configured to allow installation of the system 1 itself in a hydraulic duct 11 of a hydraulic circuit 10.

Preferably, the axial ends of the duct 3 have a cross-sectional area comprised between $18mm^2$ and $1300mm^2$, more preferably comprised between $50mm^2$ and $350mm^2$. In the case of a duct with a circular section, this corresponds to diameters substantially comprised between 5mm and 40mm, more preferably comprised between 8mm and 20mm.

Advantageously, the system 1 is particularly suitable to be installed in hydraulic circuits of devices or plants operating in the food sector, such as for example the hydraulic circuits of devices or plants for the treatment and delivery of beverages (e.g., water, beer, syrup-based beverages), or in devices operating in the medical sector, for the treatment in particular of body fluids such as blood and/or derivatives thereof.

In essence, the system 1 is usable in particular in hydraulic applications which, due to their dimensions, are not compatible with micro-fluidic analysis solutions which, as known, integrate micro-channels having dimensions in the order of 10-100 micrometres, and therefore operate with volumes of liquids in the order of microlitres or less.

Preferably, the cross-section S2 intermediate between two end cross-sections S1, S3 has a cross-sectional area A2 of lesser or greater extension than the cross-sectional area A1, A3 of each of the two end cross-sections S1, S3 by a factor comprised between 2 and 10 times, preferably comprised between 4 and 6 times.

For example, in the case of the embodiment schematically illustrated in Figure 1, 3 the end cross-sections S1, S3 have a cross-sectional area comprised between $18mm^2$ and $1300mm^2$, more preferably comprised between $50mm^2$ and $350mm^2$. In the case of a duct with a circular section, this corresponds to diameters substantially comprised between 5mm and 40mm, more preferably comprised between 8mm and 20mm. The intermediate cross-section S2 has instead a cross-sectional area comprised between $0.5mm^2$ and $80mm^2$, more preferably comprised between $0.8mm^2$ and $50mm^2$. In the case of a duct with a circular section, this corresponds to diameters substantially comprised between 0.8mm and 10.0mm, more preferably comprised between 1.0mm and 8.0mm. The duct 3 can have square or rectangular-shaped cross-sections.

Figure 7 schematically illustrates a system for measuring the degree of carbonation of water comprising a system 1 for measuring the flow according to the invention.

In particular, such a system for measuring the degree of carbonation comprises a system 1 for measuring the flow, comprising in turn the temperature sensor T and at least two pressure sensors P1 and P2 as described above, and further a flow sensor 2 which in turn may comprise at least two pressure sensors P1' and P2' arranged in a Venturi duct, i.e. in a duct having at least two different cross-sections of cross-sectional area.

A concentrated pressure drop 4 is provided between the flow measurement system 1, according to the invention, and the further flow sensor 2. Alternatively, the system 1 for measuring the flow, according to the invention, and the further flow sensor 2 are separated by a circuit segment sufficiently long to ensure a pressure drop.

The system illustrated in Figure 7 is therefore adapted to measure the amount of carbon dioxide ($CO_2$) dissolved in water by means of a carbonator device 6, i.e. to measure the degree of carbonation intended as the ratio between the amount in grams of carbon dioxide dissolved in an amount in litres of water.

This measurement is made as the difference between the flow read by the system 1 ($Q\_a$) placed in a circuit segment where the pressure of the fluid ($P\_high$) is such as to keep the carbon dioxide dissolved in the water (which may be in correspondence with the outlet of the carbonator 6), and the flow read by the flow sensor 2 ($Q\_b$) placed in a circuit segment at a lower pressure ($P\_low$) (which may be the segment adjacent to the dispenser 8) such as to achieve the formation of carbon dioxide bubbles. The aforementioned formation of carbon dioxide bubbles, which will be proportional to the degree of carbonation of the water entering the system and will occur according to Henry's law in a different manner depending on the pressure and the temperature of the fluid that are measured at the two points of interest, will cause the increase in flow in the portion of the circuit at pressure $P\_low$ compared to that at pressure $P\_high$.

The carbonation will be a function of the absolute pressures of the fluid read respectively by a pressure sensor P1 of the system 1 and by a pressure sensor P1' of the flow sensor 2, (i.e. $P\_low$ and $P\_high$), of the difference of the flow, obtainable from the system 1 and from the flow sensor 2 (i.e. the difference between $Q\_a$ and $Q\_b$) and of the temperature detected by the temperature sensor P.

The present invention also concerns a dialysis system comprising:

- a dialyser filter,
- a first system for in-line measurement of the viscosity as described above, located upstream of the dialyser filter and hydraulically connected to it, as well as
- a second system for in-line measurement of the viscosity, as described above, located downstream of the dialyser filter.

The two systems for in-line measurement of the viscosity are adapted to measure both the viscosity and the flow of the dialysis liquid entering and exiting the dialyser filter (or of the blood) and to generate respective signals indicative of the viscosity and flow.

A processing and control unit of the dialysis system is further configured to generate a performance signal of the dialyser filter on the basis of the viscosity and flow signals detected respectively downstream and upstream of the dialyser filter itself. In essence, therefore, the possibility of observing variations in the flow and viscosity of the dialysis fluid (or of the blood) at the ends of the dialyser filter allows to obtain information on the progress of the dialysis treatment performed by the dialysis system.

The possibility of detecting variations in viscosity of the dialysis fluid allows to evaluate the efficiency of the dialysis treatment. For example, the quantification of an increase in viscosity of the dialysis fluid exiting the dialyser filter compared to the dialysis fluid entering the same, allows to quantify the efficiency of disposal of the unwanted substances from the patient's blood. This information can also be used to continue or interrupt the treatment, upon reaching the desired viscosity difference values between inlet and outlet.

In practice, it has been found that the system for in-line measurement of the viscosity of a fluid, and a corresponding measurement method, according to the present invention, fulfil the task as well as the purposes set as they allow an in-line measurement of the viscosity of a fluid and also at low cost.

Another advantage of the system and of the method, according to the invention, consists in that it is possible to provide, at the firmware level, compensations for the calibration errors of the sensors, as well as compensations for the effects due to temperature variations of the fluid.

A further advantage of the system and of the method, according to the invention, consists in the fact of ensuring that, in each point of the measurement, the fluid whose viscosity is to be measured, flows only and exclusively within the duct, avoiding providing deviations of part of the flow necessary for the measurement itself.

Another advantage of the system, according to the invention, consists in the fact that the duct is easily cleanable, as the sensors used are integrated into the duct itself and/or detect the measurements of interest without direct contact with the fluid.

A further advantage of the system, according to the invention, consists in the fact that it also allows the direct measurement of the temperature of the fluid, thus avoiding making indirect measurements that in whole or in part are affected by the temperature of the wall of the duct itself.

Yet another advantage of the invention consists in the fact that it is easy to integrate into any hydraulic circuit, it being sufficient to provide a sensorised duct with the geometries described above. In addition, the possibility of using miniaturized sensors further favours the possibilities of integration into hydraulic circuits, even pre-existing ones, of various type.

Yet another advantage of the system according to the invention consists in the fact that, with a single system, the pressure, the flow, the temperature and the viscosity of a fluid can be measured simultaneously.

Yet another advantage consists in the possibility to measure the flow of a fluid regardless of its viscosity.

Yet another advantage of the invention concerns its durability, as the measurements of the parameters of interest are carried out with sensors without moving parts that, over time, can more easily deteriorate.

The system for measuring the viscosity and the flow of a fluid described above, thanks to its simplicity, its precision and the fact that it can be integrated into a disposable device, is particularly advantageous when used in the medical sector, and in particular in the hydraulic circuits where liquids of a biological nature circulate. For example, in the so-called "heart-lung" machines used for extracorporeal circulation it is possible to measure in line, and therefore to monitor in real time, the viscosity and the flow of the blood circulating in the machine with a simple and precise device and above all at low cost, replaceable from time to time at a minimum cost.

The system and the method described above also find an important application in the food sector, for example in the hydraulic circuits for the generation of beverages obtained by mixing water and syrup.

In fact, the water/syrup mixing ratios for obtaining a beverage can be varied in real time as a function of the viscosity of the syrup itself, detected in line, so as to always guarantee that a beverage with optimal organoleptic properties is obtained. Furthermore, thanks to the in-line measurement of the viscosity, it is possible to use the same syrup-based beverage dispensing device, starting from different syrups having different viscosity values, to make different beverages. In fact, the mixing parameters can be modified both based on the recipe of the beverage and based on the actual viscosity of the syrup measured in line.

Still, in the inkjet printing systems it is generally known to use additives to maintain the viscosity of the ink within certain desired values. The possibility of measuring the viscosity of the ink in line, in a simple and precise way, and at low cost, allows the aforementioned additives to be suitably dosed, in real time, in order to always keep the viscosity of the ink constant and therefore optimize the printing processes.

The system and the method thus conceived are susceptible to numerous modifications and variants all falling within the scope of the inventive concept.

Furthermore, all the details can be replaced by other technically equivalent elements. In practice, any materials can be used according to requirements, as long as they are compatible with the specific use, the dimensions and the contingent shapes.

**Claims**

1. System (1) for in-line measurement of the viscosity of a fluid flowing within a duct (3), comprising:

   - a duct (3) having three distinct cross-sections (S1, S2, S3), where said three distinct cross-sections (S1, S2, S3) are distributed along the direction of axial development of said duct (3) and where at least one cross-section (S2), intermediate between two end cross-sections (S1, S3), has a cross-sectional area (A2) of greater or lesser extension than the cross-sectional area (A1, A3) of each of said two end cross-sections (S1, S3);
   - at least three pressure sensors (P1, P2, P3) arranged in correspondence with said three distinct cross-sections (S1, S2, S3) of said duct (3) and configured to detect the instantaneous pressure of said fluid in correspondence with said three distinct cross-sections (S1, S2, S3) of said duct (3).

2. System (1), according to claim 1, wherein said end cross-sections (S1, S3) have cross-sectional areas (A1, A3) of equal extension.

3. System (1), according to one or more of the preceding claims, wherein each of said pressure sensors (P1, P2, P3) comprises a sensing element (21, 22, 23) configured to face the internal volume of said duct (3) and to be in direct contact with said fluid, said sensing element (21, 22, 23) being integrated into a wall (30) of said duct (3).

4. System (1), according to claim 3, wherein said sensing element (21, 22, 23) is positioned flush with the inner face of said wall (30) of said duct (3).

5. System (1), according to one or more of the preceding claims, comprising a temperature sensor (T) configured to detect the instantaneous temperature of said fluid flowing in said duct (3).

6. System (1), according to claim 5 wherein said temperature sensor (T) is an infrared sensor, said temperature sensor (T) being associated with a wall (30) of said duct (3), said system (1) comprising at least one body (31B) transparent to infrared rays associated with said wall (30) and interposed between said temperature sensor (T) and the internal volume of said duct (3).

7. System (1), according to one or more of the preceding claims, **characterized in that** it is integrated into a disposable device.

8. System (1) according to one or more of the preceding claims, wherein said duct (3) comprises, at its axial ends, respective fittings (12) configured to allow installation of said system (1) in a hydraulic duct (11) of a hydraulic circuit (10).

9. System (1), according to one or more of the preceding claims, wherein said duct (3) has, in sequence:

  - at least a first segment (T1) having substantially constant cross-section (S1);
  - at least a second segment (T2) having substantially constant cross-section (S2);
  - at least a third segment (T3) having substantially constant cross-section (S3);

said segments (T1, T2, T3) with a substantially constant cross-section (S1, S2, S3) being connected by segments with an increasing or decreasing cross-section.

10. Dialysis system comprising a dialyser filter, a first system for in-line measurement of the viscosity (1) according to one or more of the preceding claims, and a second system for in-line measurement of the viscosity (1) according to one or more of the preceding claims, said first system (1) being hydraulically connected to said dialyser filter upstream thereof, said second system (1) being hydraulically connected to said dialyser filter downstream thereof, said first system (1) and said second system (1) being adapted to measure the viscosity and the flow of a dialysis liquid and/or of blood respectively entering and exiting said dialyser filter and to generate respective signals indicative of the viscosity and flow, said dialysis system comprising a processing and control unit configured to receive and process said signals indicative of the viscosity and flow detected upstream and downstream of said dialyser filter and to generate a signal indicative of the progress of the treatment performed by said dialysis system on the basis of said signals indicative of viscosity and flow.

11. Method for in-line measurement of the viscosity of a fluid flowing within a duct (3), comprising the steps of:

  - detecting the instantaneous pressure of said fluid in correspondence with three distinct cross-sections (S1, S2, S3) of said duct (3) where said three distinct cross-sections (S1, S2, S3) are distributed along the direction of axial development of said duct (3) and where at least one cross-section (S2) intermediate between two end cross-sections (S1, S3) has a cross-sectional area (A2) of greater or lesser extension than the cross-sectional area (A1, A3) of each of said two end cross-sections (S1, S3);
  - calculating the viscosity of said fluid on the basis of the instantaneous pressure of said fluid detected in correspondence with said three distinct cross-sections (S1, S2, S3) of said duct (3).

12. Method for in-line measurement of the viscosity of a fluid according to the preceding claim, wherein said step of calculating the viscosity of said fluid comprises the step of solving the system of equations

$$p_1 - p_2 = \frac{1}{2}\rho\left(1 - \frac{A1}{A2}\right)v_1^2 - \Delta p_{losses}(v_1, \mu)$$

and $p_1 - p_3 = \Delta p_{losses}(v_1, \mu)$ where

- $p_1$, $p_2$ and $p_3$ are the pressures detected by the pressure sensors (P1, P2, P3) respectively,
- $\rho$ is the density of the fluid,
- A1 is the area of each of said two end cross-sections (S1, S3),
- A2 is the area of said intermediate cross-section (S2),
- $v_1$ is the speed of said fluid in correspondence with each of said two end cross-sections (S1, S3),
and where $\Delta p_{losses} = f(\mu, v_1, geometry)$, i.e. the pressure loss $\Delta p_{losses}$ is a function of the dynamic viscosity $\mu$, of said fluid and of the geometry of said duct (3), where said geometry is known;
the unknowns of said system of two equations being $v_1$ and the viscosity $\mu$, which are therefore uniquely determinable.

13. Method for in-line measurement of the viscosity of a fluid according to claim 11 or 12, comprising the step of performing a self-calibration procedure of said pressure sensors (P1, P2, P3), said self-calibration procedure comprising the step of calculating a correction factor ($\Delta p_{corr}$) of the pressure difference detected between pairs of said pressure sensors (P1, P2, P3) at zero fluid speed.

14. System (1) for in-line measurement of the flow of a fluid flowing within a duct (3), comprising:

- a duct (3) having at least two distinct cross-sections (S1, S2) distributed along the direction of axial development of said duct (3) and where at least a first cross-section (S1) has a cross-sectional area (A1) of greater or lesser extension, preferably greater, than the cross-sectional area (A2) of the second cross-section (S2) downstream of said first cross-section (S1);
- at least two pressure sensors (P1, P2) arranged in correspondence with said two distinct cross-sections (S1, S2) of said duct (3) and configured to detect the instantaneous pressure of said fluid in correspondence with said two distinct cross-sections (S1, S2) of said duct (3);
- at least one temperature sensor (T) configured to detect the instantaneous temperature of said fluid flowing in said duct (3).

15. Method for in-line measurement of the flow of a fluid flowing within a duct (3), comprising the steps of:

- detecting the instantaneous pressure of said fluid in correspondence with at least two distinct cross-sections (S1, S2) of said duct (3) distributed along the direction of axial development of said duct (3), where at least a first cross-section (S1) has a cross-sectional area (A1) of greater or lesser extension, preferably greater, than the cross-sectional area (A2) of a second cross-section (S2) downstream of said first cross-section (S1);
- detecting the instantaneous temperature of said fluid;
- calculating the flow of said fluid on the basis of the instantaneous pressure of said fluid detected in correspondence with said two distinct cross-sections (S1, S2) of said duct (3) and on the basis of said instantaneous temperature, said step of calculating the flow of said fluid using said instantaneous temperature of said fluid to compensate for the effects due to variations in temperature and viscosity of said fluid, the viscosity-temperature curve of said fluid being known.

Fig. 1

Fig. 2

Fig. 3

— N/A

EP 4 372 323 A1

Fig. 4

Fig. 5

Fig. 6

Fig. 7

13

Europäisches
Patentamt

European
Patent Office

Office européen
des brevets

# EUROPEAN SEARCH REPORT

Application Number

EP 23 21 0565

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/134765 A1 (RHEOSENSE INC [US]) 12 June 2008 (2008-06-12) | 1-9, 11-15 | INV.<br>G01F1/36 |
| A | * paragraphs [0003], [0009], [0010], [0034], [0036], [0042], [0043]; claim 10; figures 1,5 12A-12C * | 10 | G01F1/44<br>G01N11/08<br>G01F1/88<br>G01F15/02 |
|  | ----- |  | G01F1/50 |
| X | US 6 386 016 B1 (GLEISSLE WOLFGANG [DE]) 14 May 2002 (2002-05-14) | 1-3 | G01J5/04<br>G01J5/0875 |
| A | * column 2, line 61 - line 66; claim 1; figures 1,2 * | 4-15 | A61M1/14<br>A61M60/37 |
|  | ----- |  |  |
| X | US 6 463 810 B1 (LIU TAY-JIAN [TW]) 15 October 2002 (2002-10-15) | 14,15 | ADD.<br>G01N11/00 |
| A | * column 1, line 12; figure 2 *<br>* column 6, line 52 - line 67 * | 1-13 |  |
|  | ----- |  |  |
| A | EP 3 261 708 B1 (MAQUET CARDIOPULMONARY GMBH [DE]) 4 May 2022 (2022-05-04)<br>* paragraphs [0016], [0018], [0025], [0047], [0050] - [0051], [0058] * | 6,10 |  |
|  | ----- |  | TECHNICAL FIELDS SEARCHED (IPC)<br><br>G01F<br>G01N<br>G01J<br>A61M |

The present search report has been drawn up for all claims

| Place of search | Date of completion of the search | Examiner |
|---|---|---|
| The Hague | 4 April 2024 | Geromel Prette, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 23 21 0565

This annex lists the patent family members relating to the patent documents cited in the above-mentioned European search report.
The members are as contained in the European Patent Office EDP file on
The European Patent Office is in no way liable for these particulars which are merely given for the purpose of information.

04-04-2024

| Patent document cited in search report | | Publication date | Patent family member(s) | | Publication date |
|---|---|---|---|---|---|
| US 2008134765 | A1 | 12-06-2008 | US | 2008134765 A1 | 12-06-2008 |
| | | | WO | 2009061943 A2 | 14-05-2009 |
| US 6386016 | B1 | 14-05-2002 | DE | 19848687 A1 | 27-04-2000 |
| | | | US | 6386016 B1 | 14-05-2002 |
| US 6463810 | B1 | 15-10-2002 | TW | 421710 B | 11-02-2001 |
| | | | US | 6463810 B1 | 15-10-2002 |
| EP 3261708 | B1 | 04-05-2022 | CN | 107530530 A | 02-01-2018 |
| | | | CN | 112370593 A | 19-02-2021 |
| | | | EP | 3261708 A1 | 03-01-2018 |
| | | | JP | 6827634 B2 | 10-02-2021 |
| | | | JP | 6919022 B2 | 11-08-2021 |
| | | | JP | 2018512576 A | 17-05-2018 |
| | | | JP | 2020144145 A | 10-09-2020 |
| | | | KR | 20180026364 A | 12-03-2018 |
| | | | US | 2018110913 A1 | 26-04-2018 |
| | | | US | 2020164132 A1 | 28-05-2020 |
| | | | WO | 2016138380 A1 | 01-09-2016 |

EPO FORM P0459

For more details about this annex : see Official Journal of the European Patent Office, No. 12/82